# EUROPEAN PATENT APPLICATION

(11) **EP 1 013 283 A2**
(43) Date of publication of application: **28.06.2000**
(21) Application number: 00200143.6
(22) Date of filing: 01.04.1993
(51) Int. Cl.: A61K 38/40, A23J 3/04, A23K 1/17, A23L 1/03, A61P 31/00

(54) **Pharmaceutical and foodstuff compositions containing transferrins and antibacteral agents for potentiating host defence activity and the treatment of infections**

(30) Priority: 02.04.1992 JP 15262092
(62) Divisional of application: 93302568.6
(71) Applicant: IMMUNO JAPAN INC., Suginami-ku, Tokyo 167 (JP)
(72) Inventor: The designation of the inventor has not yet been filed
(74) Representative: Grant, Anne Rosemary

(57) **Abstract**

When the lactoferrin family protein is orally administered to mammals, the phagocytic activity of the mammals is significantly potentiated through host-mediated mechanism, resulting in (1) enhancement in resistance against infection by pathogenic microorganisms, and (2) significant potentiation of the efficacy of bacteriostatic antimicrobial agents. The lactoferrin family protein is very useful for treating and/or preventing infectious diseases, especially those caused by opportunistic pathogens.

## Description

### Background and Introduction

The present invention relates to (1) formulated medicines or processed foods containing protein class lactoferrin family as active ingredient together with a bacteriostatic antibiotic, and (2) the method of preventing and/or treating infectious diseases in mammals caused by opportunistic pathogens by using either the medicines or the foods or both which contain lactoferrin and a bacteriostatic antibiotic.

Infectious diseases caused by bacterial pathogens have long been the leading cause of death in human beings. However, even though remarkable advances in chemotherapies, including both antibiotics and synthetic organic chemotherapies, have greatly reduced deaths caused by infectious diseases, people appear to think that such diseases have been completely overcome. Infectious diseases are still one of the most serious causes of death, especially in developing countries. The spread of infectious disease can rapidly become serious and even fatal when the host-defense capability is severely impaired, for example by aging, surgical operation, cancer, immuno-suppressant therapies, diabetes mellitus and other conditions.

One example of the above is that most patients with cancer die because of infectious diseases, not due to the cancer per se. The causative agents are in most cases the so called opportunistic pathogens that are weakly virulent in normal healthy people. Though it is rare that opportunistic pathogens cause serious infection in healthy mammals, such hosts cannot protect themselves from the attack of opportunistic pathogens when their host-defense capability is severely impaired. Those who have impaired host-defense capability are called compromised. It is also difficult for elderly people to recover from opportunistic infections in the respiratory tract, especially those elderly people who are bed ridden since even high dosages of antibiotics cannot save the patients because of the impairment in host defense capability.

Another example is post-operative infections, especially after abdominal or breast operations, where several of the patients become infected with opportunistic pathogens in spite of the advances in chemotherapies and improved facilities such as clean rooms. The reason for such infections is that the surgical operation inevitably impairs the host-defense system even if the host-defense system is normal before the operation.

When leukemia patients are treated with anti-leukemia chemotherapies, the treatment greatly reduces the leukocyte count and the patients become highly susceptible to the attack of opportunistic pathogens. Therefore, treatment is started after eliminating pathogens from the body surface and digestive tract while isolating the patient in a germ-free environment. However, infectious diseases still occur (probably due to intestinal flora) in one hundred percent of such patients in spite of the very intensive care.

Infectious diseases occurring in such compromised hosts are characterized as follows: (1) in most cases the causative agents are opportunistic pathogens less virulent in healthy mammals, and (2) the infections get more serious earlier and antibiotics are less effective.

The mechanism of the so called "compromised condition" has not yet been scientifically clarified. There are two systems in the host-defense mechanism: (1) cellular immunity represented by lymphocytes and phagocytes such as neutrophils or macrophages, and (2) humoral immunity consisting of antibodies and complement. The two immune systems work in a complex, concerted manner thereby protecting mammals from infection by pathogens. It is said that the most important roles are played by phagocytes and humoral factors in the prevention of infections by pathogenic microorganisms.

Apart from the patients who received antitumor chemotherapies which result in greatly reduced blood leukocyte levels, even the elderly bed ridden have normal numbers of peripheral leukocytes and normal levels of humoral immune factors as compared with healthy persons. Therefore, the impairment in the host-defense capability in compromised hosts is likely attributable to a functional defect but not in the decrease of host defensive factors.

Consequently, massive treatment with antimicrobial therapies is certainly effective in only the partial prevention and treatment of infectious diseases caused by opportunistic pathogens.

The inventors have studied the treatment of infectious disease in compromised hosts for years and have found that (1) onset of infectious diseases by opportunistic pathogens closely correlates to phagocytic activity in the hosts, (2) oral administration of the class lactoferrin protein family increases phagocytic activity at the whole animal level, and (3) potentiation of phagocyte activity is very useful for treatment and prevention of infectious diseases. Thus, the inventors completed this invention.

### Summary of the Invention

An object of the present invention is to provide remedies to potentiate the host-defense capability in compromised hosts by enhancing phagocytic activity, thus solving fundamental disorders underlying the compromised state.

Another object is to provide a pharmaceutical composition for treatment and/or prevention of opportunistic infectious diseases in mammals and for enhancing the efficacy of the host immune system in a mammal in need thereof which contains (a) proteins belonging to the class of mammalian transferrin/lactoferrin family, (b) a bacteriostatic antibiotic, and (c) optionally a pharmaceutically acceptable carrier. The composition can be in the form of a tablet or a hard gelatin capsule.

Another object is to provide method for the prevention and/or treatment of opportunistic disease which involves administering to a mammal in need thereof an amount of the pharmaceutical composition described above, said amount effective to prevent and/or treat said opportunistic disease caused by opportunistic microorganisms.

Yet another object is to provide a method of potentiating the host immune system in a mammal in need thereof which involves administering to a mammal in need thereof an amount of the pharmaceutical composition described above, said amount effective to prevent and/or treat said opportunistic disease caused by opportunistic microorganisms.

Another object is to provide a solid or liquid food product which comprises food and an amount of the pharmaceutical composition described above effective to prevent and/or treat opportunistic infections caused by opportunistic microorganisms.

### Brief Description of the Drawings

Figures 1-4 show the serum antibiotic concentrations with or without lactoferrin administration, the open circles are the lactoferrin group and the closed circles are the controls.

Figure 1 shows the serum concentrations of cephalexin.

Figure 2 shows the serum concentrations of cefixime.

Figure 3 shows the serum concentration of cefaclor.

Figure 4 shows the serum concentrations of cefroxadine.

Figure 5 shows the viable cell count in the incubation mixture containing mouse neutrophils and *Escherichia coli c* 11 at a rate of 1:1. The closed circles are controls without neutrophils, the open circles neutrophil controls, the closed squares lactoferrin at a concentration of 10µ/ml, and the open squares lactoferrin at a concentration of 1,000 µ/ml.

Figure 6 shows the effect of lactoferrin treatment on phagocytic activity of whole blood from mice. The closed circles represent the control group without neutrophils, the open circles the control group of neutrophils without lactoferrin treatment, and the open squares the lactoferrin group.

### Detailed Description of the Invention

The present invention provides formulated medicines containing the class lactoferrin as an active ingredient to potentiate host-defense capability, the formulated medicines containing lactoferrin as an active ingredient either to treat and/or to prevent infectious diseases, and the processed foods containing lactoferrin at doses of more than 150 mg/kg body weight to potentiate host-defense capability.

The lactoferrin used in the present invention belongs to the protein class of lactoferrin family recovered from the milk of humans, cows, goats and sheep, and the activity is independent of the degree of iron saturation.

The protein is not only used solely for the treatment and/or prevention of infectious diseases, but also used in combination either with antibiotics or antimicrobial chemotherapies.

The protein class of lactoferrin family used in the present invention are those obtained from mammalian milk, hen egg white, or leukocytes. Methods for isolation and purification of these proteins are well known to those skilled in the art and are described in detail in papers and books which are readily available.

The proteins are basic glycoproteins having a molecular weight of 70-90 kD that can form complexes by chelating to Fe³⁺. Since such proteins as transferrin found in blood, lactoferrin found in milk, and ovotransferrin found in avian albumen are thought to be relatively originating from the same ancestor protein, they are classified into the class lactoferrin family. However, the role of lactoferrin is not clear in spite of the wide distribution.

The inventors studied *in vitro* antimicrobial activity of protein class lactoferrin family in natural form using nutrient broth agar plate as the medium. However, these proteins could not prevent the growth of pathogenic microorganisms when the concentrations were increased to 1000µg/ml; the medium used contains sufficient amounts of iron. Similar results were obtained using apo-lactoferrin (or iron-free lactoferrin) through holo-lactoferrin (or iron saturated lactoferrin)(Example 1). The results suggest protein class lactoferrin families have no direct inhibitory activity against pathogenic microorganisms even *in vivo.*

Surprisingly, oral administration of bovine lactoferrin synergistically potentiated the efficacy of β-lactam antibiotics by 2-5 times in an acute septic model when administered simultaneously with antibiotics. In these experiments the mice were intraperitoneally infected with *Klebsiella pneumoniae* 3k25 followed by oral administration of the antibiotics and lactoferrin one hour later. Moreover, similar results were obtained using other gram negative bacteria, e.g., *E. coli, Proteus, Klebsiella,* and gram positive bacteria, e.g. *Staphylococcus aureus* and *Streptococcus pneumoniae.* (Examples 3 and 4)

As the next step, the effect of lactoferrin on the efficacy of injectable β-lactam antibiotics was studied with the use of the same experimental system as described above. It was observed that simultaneous treatment with oral lactoferrin potentiated the efficacy of the antibiotics 2 to 5 times, as compared with the antibiotics alone. A remarkable feature found in these studies is that the activity potentiated due to oral lactoferrin therapy is only seen in the combination of lactoferrin with any bacteriostatic antibiotics because the efficacy potentiated was observed not only in the combination of lactoferrin with cephalosporins, but also with penicillins, e.g., ampicillin, tetracyclines, macrolides, lincomycins and chloramphenicols. It is expected that any bacteriostatic can be utilized with lactoferrin.

Protein class lactoferrin family potentiates the efficacy of bacteriostatic antibiotics but it cannot affect the efficacy of bactericidal antibiotics, e.g., aminoglycosides and guinolones (Example 5). This fact suggests why a synergetic effect was never seen when lactoferrin was administered simultaneously with bactericidal chemotherapies, the microorganisms will inevitably be killed in the case of bactericidal chemotherapies when the concentration inside the cells reaches given certain levels. In contrast, bacteriostatic antibiotics do not work bactericidally even if the concentrations reach high levels *in vivo;* the pathogens resume proliferation when the concentrations gradually decrease as they are excreted from the body. Lactoferrin enhances the nonspecific defense system through host-mediated mechanism eliminating pathogens much faster.

Interestingly, as shown in Table 6, oral treatment with lactoferrin inhibits colonization by *Proteus mirabilis* 1287 in the bladder, indicating that the treatment is effective on chronic renal infection caused by an opportunistic pathogen. This inhibitory effect of lactoferrin is seen without simultaneous administration of antimicrobial chemotherapies, but the inhibitory activity is synergistically enhanced by simultaneous administration of antimicrobial chemotherapies that are excreted through the renal route. Although oral lactoferrin therapy cannot save infected mice from death in the case of an acute septic model (intraperitoneal injection of pathogenic bacteria), the treatment with lactoferrin alone shows significant beneficial effects on such chronic infectious models as urinary tract infection. This fact indicates that oral lactoferrin therapy is highly beneficial for treatment of chronic infectious diseases caused by opportunistic pathogens in compromised hosts since most chronically persisting refractory infections are caused by opportunistic pathogens in immune-compromised hosts.

The minimum effective dose of protein class lactoferrin family ranges from 0.1-250 mg per kg body weight depending on animal models and general appropriate doses are 1-50 mg/kg for the treatment and/or prevention of infectious diseases. It is desirable to treat through the oral route to avoid immunological sensitization by foreign antigens, but other routes such as through the skin or mucus membrane can be used. The reasons why lactoferrin is highly effective in the treatment of infectious diseases in such a small dose are (1) it is hardly digestible in gastrointestinal tract, (2) lactoferrin exerts its effects through binding with receptors present on intestinal mucous, and (3) once the receptors are saturated with lactoferrin, the potentiating effect on host defense system becomes maximal.

In order to establish the optimal dose-schedule, lactoferrin was administered once to mice either on day 5, 3, 2 or 1 before intraperitoneal challenge by *Klebsiella pneumoniae,* the mice were treated with oral cephalexin one hour after the injection. Consequently, it is revealed that the previous treatment with oral lactoferrin less than 24 hours prior to the injection significantly enhances the antibiotic efficacy, but when lactoferrin is given more than 48 hours before injection it no longer enhances the activity of cephalexin. This fact indicates that once lactoferrin binds to its intestinal receptors the stimulatory effect to the local immune system persists for 24 hours, but the effect gradually decays as the time elapses more than 24 hours. It is known that neutrophils plays the major role through phagocytosis in killing invading bacterial pathogens in the living body, but the half life is as short as 6-12 hours. In view of the persistence of the activated state, the duration of neutrophils is clearly shorter than the macrophages that persists in an activated state for longer than a week.

On the other hand, the present inventors confirmed that oral lactoferrin treatment never accelerates intestinal absorption of ingested antibiotics. As shown in experiment 2, when mice were orally administered lactoferrin simultaneously with four kinds of cephalosporin antibiotics (cephalexin, cefaclor, cephroxazine, or cefixime) and the blood was withdrawn periodically to determine blood levels of the antibiotics, the levels were virtually the same in each instance, indicating that lactoferrin has no effect on the intestinal absorption of β-lactam antibiotics.

When protein class lactoferrin family is parenterally injected into the animals with infectious diseases simultaneously with β-lactam antibiotics, it significantly enhances the antibiotic efficacy *in vivo.* This fact strongly supports the hypothesized mode of action described above. In addition, neutrophils were collected from the blood of mice and (1) the phagocytic activity and (2) the generation of chemiluminescence was determined in the presence of lactoferrin. However, lactoferrin showed no stimulatory effects when incubated with neutrophils *in vitro.* Also, the neutrophils collected from the peritoneal exudate by injecting starch intraperitoneally were not activated by lactoferrin because neither (1) phagocyte activity nor (2) generation of oxygen radicals was unaffected in the presence of lactoferrin *in vitro.* All of these facts indicate that the mode of action of lactoferrin is host-mediated activation of the defense system.

In contrast, the neutrophils collected from the mice that received oral treatment with protein class lactoferrin family showed much more phagocyte activity than those from untreated controls and generated significantly more oxygen radicals than the controls. Thus, evidently oral treatment with lactoferrin affects the host-defense system by activating neutrophil functions thereby protecting the host from attack by pathogenic microorganisms. The effectiveness of the oral route suggests that lactoferrin is promising as a food additive in protecting animals from infection by pathogenic microorganisms.

To prepare the pharmaceutical preparation containing protein class lactoferrin family, it is important to avoid protein denaturation. Lactoferrin is compatible with such ordinary pharmaceutical additives as glucose, maltose, sorbitol, dextrin, starch, microcrystalline cellulose, hydroxypropyl-cellulose, carboxymethyl-cellulose, talc, either magnesium or calcium stearate, and the granules and the tablets can readily be prepared when mixed with these inactive additives followed by the appropriate mechanical means. The liquid forms also can be made by dissolving it with preservatives.

In order to prepare the foods containing sufficient amounts of lactoferrin, the admixtures with supplemented milk, yogurt, skim milk powder, lactic acid bacteria fermented milk, chocolate, tablet sweets, and powdered beverages are processed by appropriate means. The most important factor is to avoid protein denaturation during processing by not exceeding a temperature of about 60°C. The lactoferrin contents in the processed foods should be more than 150 mg/kg in solid foods and more than 30mg/200 ml of liquid foods and desirable amounts should be 250 mg/kg food and 50 mg/200 ml liquid. The lactoferrin content in mature raw bovine milk is 60-150 mg/kg.

### Examples

Example 1-powdered bovine lactoferrin 200 g prepared by lyophilization was sieved through 0.8 mm mesh and mixed with calcium biphosphate 60 g, magnesium stearate, silica gel 1g (Degussa, Aerosil 200), α-starch 40 g. The mixture, 150 mg, was ascetically filled into a hard gelatin capsule #3. This capsule contains 100 mg of lactoferrin per capsule.

Example 2-Lyophilized lactoferrin powder 45 g, was mixed with sucrose 80 g, lactose 100 g, and deionized water was sprayed onto the mixture while mixing. Then the mixture was granulated by a vertical granulator. The granules were dried below 45°C. Other granules were made by mixing sucrose 310 g, ascorbic acid 75 g, lemon essence 0.1 ml and deionized water 80 ml, and the wet mixture was granulated by passing through 0.8 mm mesh. The resulting two kinds of granules were mixed with α-starch 65 g, and tabulated as a circular tablet weighing 1.8 g each. This tablet contains 120 mg lactoferrin per tablet corresponding to 67 g lactoferrin per kg of tablet.

Example 3-groups of six male mice strain ddY, four weeks old, were used in this experiment. They were intraperitoneally infected with 3% mucin suspension of *Escherichia coli* strain c-11 at a dose of 1 X 10⁶ cells per mouse. Oral cephalosporin antibiotic was administered one hour after the injection simultaneously with bovine milk lactoferrin (26% iron saturation), 1 mg/mouse. Each control was given cephalosporin alone. The mice were observed one week after the injection and survivors were regarded as completely cured. As shown in Table 1, the ED50s were reduced by simultaneous administration of lactoferrin to 1/4 in the case of cephalexin, to 1/4 in the case of cephixime, to 1/3 in the case of cefroxazine, and to 1/2 in the case of cefaclor. Therefore, the simultaneous administration with cephalosporin antibiotics greatly potentiates the efficacy against *Escherichia coli* infection.

**Table 1.**

| Synergetic effect of lactoferrin and β-lactam antibiotics against *E. coli* | | |
|---|---|---|
| β-lactam antibiotics | Lactoferrin | ED50(mg/mouse) |
| cefalexin | - | 8.54 |
| | + | 1.42 |
| cefixime | - | 1.03 |
| | + | 0.23 |
| cefaclor | - | 0.75 |
| | + | 0.38 |
| cefroxadine | - | 1.27 |
| | + | 0.42 |

Example 4-The efficacy potentiation of lactoferrin was examined using mice infected with *Proteus vulgaris* OX.8. Each group of six mice, strain ddY, four weeks old, were intraperitoneally infected with P. *vulgaris* suspended in 3% gastric mucin at a dose of 2 x 10⁶ cells per mouse. One hour after the injection, lactoferrin (0.5 mg/mouse) was orally given simultaneously with cephalosporins. The mice were observed for one week after the injection and the survivors were regarded as completely cured.

**Table 2.**

| Synergetic effect of lactoferrin and β-lactam antibiotics against *Proteus* | | |
|---|---|---|
| β-lactam antibiotics | Lactoferrin | ED50(mg/mouse) |
| cefalexin | - | 0.402 |
| | + | 0.185 |
| cefixime | - | 0.519 |
| | + | 0.177 |
| cefroxadine | - | 0.606 |
| | + | 0.312 |
| cefaclor | - | 1.790 |
| | + | 0.058 |
| ampicillin | - | 0.300 |
| | + | 0.148 |

As shown in Table 2, the simultaneous administration with lactoferrin reduced the ED50s to 1/2 in cephalexin, to 1/3 in cephixime, to 1/3 in cefaclor and to 1/2 in ampicillin. Consequently, the efficacy potentiation of cephalosporin due to simultaneous treatment with lactoferrin is not restricted merely to the pathogens belonging to the genus *Escherichia* and Klebsiella, but also includes Proteus.

Example 5-The antimicrobial activities of chemotherapies were compared with or without lactoferrin treatment. The antimicrobial agents used were new quinolones, aminoglycoside antibiotic, gentamicin, erythromycin, chloramphenicol and minocycline. The efficacy was compared between the controls and lactoferrin groups. Mice strain ddY, four weeks old, were randomly allocated into groups (n=6) and intraperitoneally infected with *Escherichia coli c* 11 at a dose of 1 x 10⁶ cells per mouse. One hour after the injection the chemotherapies and lactoferrin (0.5 mg/mouse) were simultaneously given orally except gentamicin was given subcutaneously since it is unabsorable from the intestinal tract. Evaluation of potentiation of the chemotherapies by lactoferrin is the same as described above. As shown in Table 3, lactoferrin again potentiated the efficacy of bacteriostatic chemotherapies such as erythromycin, chloramphenicol, tetracycline *in vivo,* but not bactericidal chemotherapies, new quinolones and gentamicin. The efficacy potentiations due to lactoferrin were three times higher than chemotherapies alone.

**Table 3.**

| Synergetic effect of lactoferrin and synthetic antibiotics, aminoglycoside antibiotics, tetracycline and chloramphenicol | | |
|---|---|---|
| β-lactam antibiotics | Lactoferrin | ED50(mg/mouse) |
| ofloxacin | - | 0.231 |
| | + | 0.251 |
| ciprofloxacin | - | 0.200 |
| | + | 0.214 |
| tosfloxacin | - | 0.013 |
| | + | 0.009 |
| gentamicin | - | 0.608 |
| | + | 0.610 |
| minocycline | - | 1.859 |
| | + | 0.631 |
| chloramphenicol | - | 5.11 |
| | + | 1.94 |

The results suggest that oral treatment with lactoferrin is able to potentiate antimicrobial efficacy of such bacteriostatic chemotherapies as β-lactams, macrolides and tetracyclines antibiotics and sulfonamides, but it did not show synergistic actions with those antibiotics which are bactericidal *in vivo.* This fact indicates that immunological surveillance by the host-defense system can readily recognize antigenically modified but still living bacteria due to the action of chemotherapies.

Example 6-Optimal dose of lactoferrin was studied using mice strain ddY (n=6), four weeks old. The mice were intraperitoneally infected with *Escherichia coli c* 11 at a dose of 1 x 10⁶ cells per mouse, and they were treated once orally with a mixture of lactoferrin and cefaclor one hour after the injection.

The survivors one week after injection were regarded as completely cured, and the results are shown in Table 4. Lactoferrin showed the same potentiating activity from 0.05 to 50 mg/kg of body weight on the efficacy of cefaclor, indicating the characteristic for the mode of action of lactoferrin: that is, once lactoferrin binds to the receptors present on the surface of intestinal mucus membranes, the binding stimulates the host-defense system and when the receptors are saturated with lactoferrin, the response reaches a maximum.

**Table 4.**

| Effect of lactoferrin dosage on antibiotic activity | |
|---|---|
| Lactoferrin | ED50 (mg/mouse) |
| 50.0 | 0.503 |
| 5.0 | 0.503 |
| 0.5 | 0.503 |
| 0.05 | 0.503 |
| 0.005 | 0.755 |
| 0.000 | 1.510 |

Example 7-The timing of lactoferrin administration is very important not only from a clinical point of view, but also from an economical point of view, since it indicates whether or not the treatment with lactoferrin is prophylactic, or curative, or both. The timing was studied using the same model as described in Example 6. Cefaclor was orally given one hour after injection of the bacteria. Bovine native lactoferrin orally administered once at a dose of 0.5 mg/mouse on day 5, 3, 2 before injection was completely ineffective, and the group dosed on day 1 before injection was less effective, as compared with the group dosed one hour after injection. The results indicate that the potentiating effect of lactoferrin rapidly disappears within 24 hours, coinciding with the half life of neutrophils.

**Table 5.**

| Effect of administration time of lactoferrin on antibiotic activity | |
|---|---|
| Administration time of Lactoferrin (day) | ED50 (mg/mouse) |
| -5 | 0.992 |
| -3 | 0.787 |
| -2 | 0.625 |
| -1 | 0.393 |
| 0 | 0.312 |
| control | 1.846 |

Experimental Example 1-The minimum inhibitory activity of bovine milk lactoferrin was determined using agar-dilution methods. The medium used was nutrient broth agar and the inoculum size was 10⁴ colony forming units per ml. Lactoferrins used were bovine native, holo- and apo-lactoferrins. The native, holo- and apo-lactoferrins showed no growth-inhibitory activity at a concentration of 4,000 mg/ml. The bacteria used in this experiment were *Staphylococcus aureus* 209P, *S. aureus* Smith, *S. aureus* 227 (MRSA), *S. epidermidis* ATCC 13228, *Micrococcus luteus* ATCC 9341, *Bacillus subtilis* ATCC 6633, *Enterococcus faecalis* 64, *E. faecalis* 95-1, *Escherichia coli* NIHJ JC-2, *E. coli* c-11, *E. coli* #35, *Klebsiella pneumoniae* ATCC 10031, *K. pneumoniae* 3k-25, *K. pneumoniae* IFO 3512, *K. oxytoca* 1006, *Shigella sonnei, S. flexineri, Salmonella typhi, S. paratyphi* A.S. *paratyphi* B, *Vibrio cholerae* 569B, *Proteus mirabilis* GN79, *P. mirabilis* 1287, *P. vulgaris* IFO 3851, *P. vulgaris* GN76, P. *morganii* IFO 3848.

Experimental Example 2-The effect of lactoferrin on intestinal absorption of cephalosporin antibiotics was studied using male ddY mice (n=6), five weeks old.

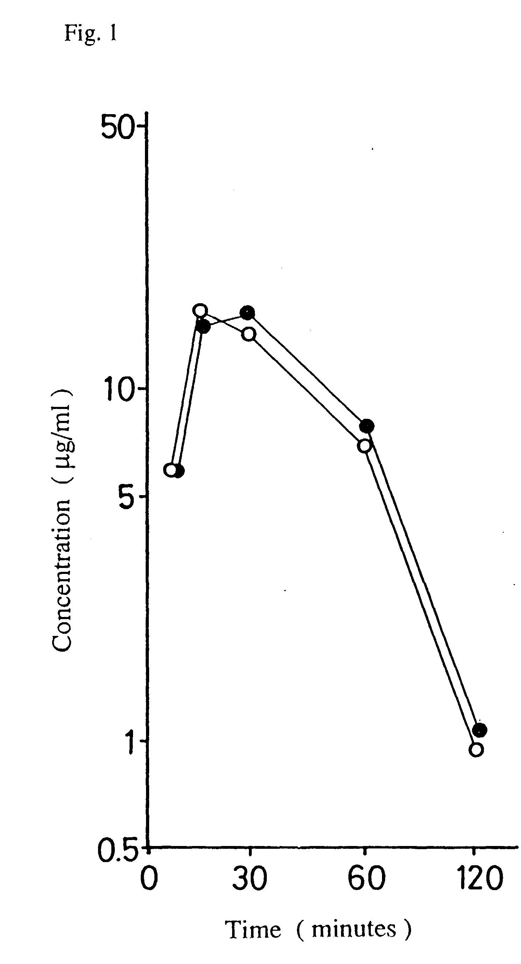

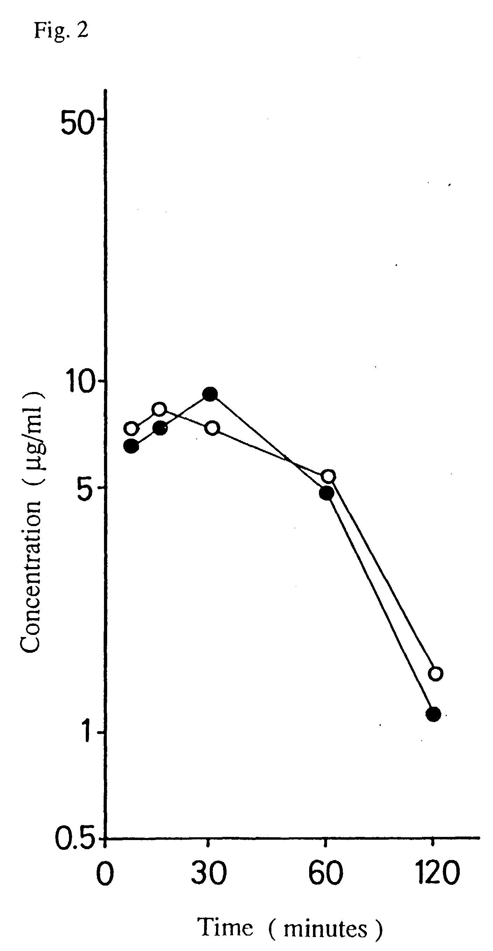

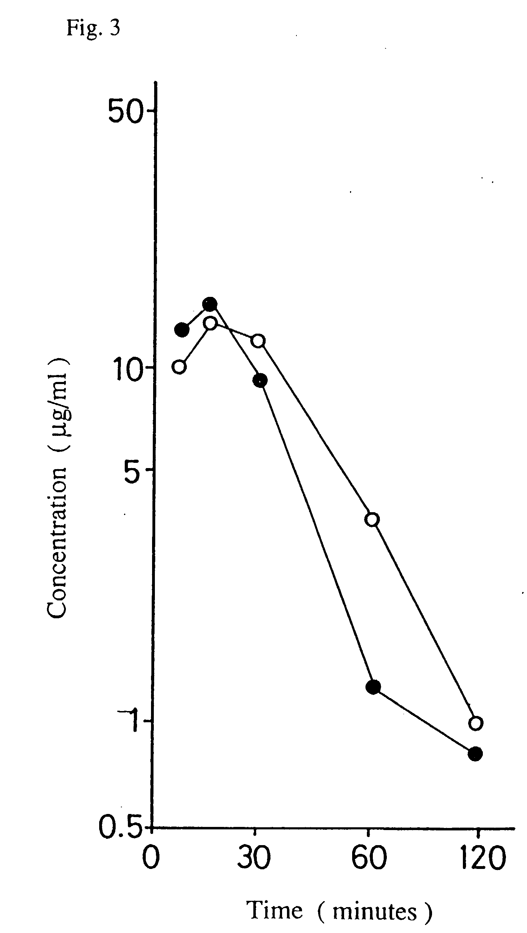

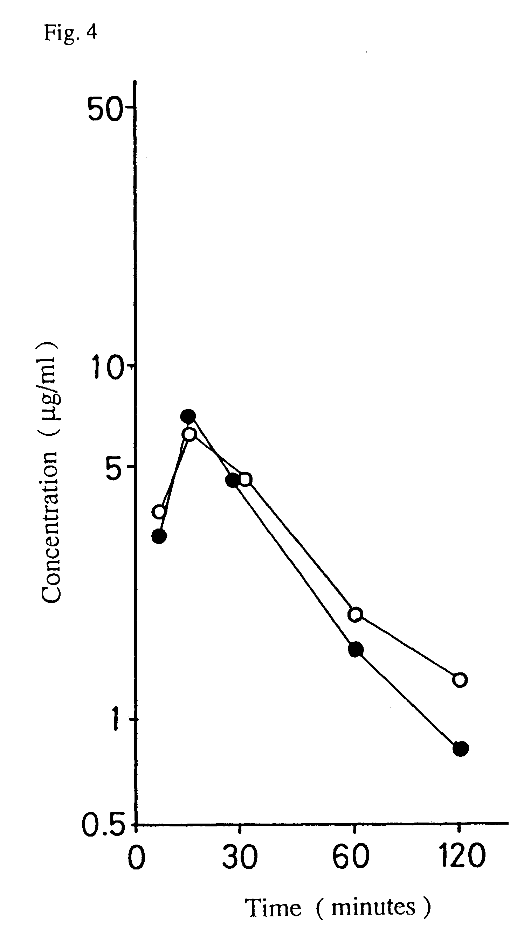

The cephalosporin antibiotics used were cephalexin, cefixime, cefaclor and cefroxazine, and the serum , concentrations were determined by bioassay using *Bacillus subtilis.* The doses of the antibiotics and lactoferrin were 0.5 mg per mouse of each. As shown in Figures 1-4, the simultaneous administration of lactoferrin had no effect on the serum concentrations of the antibiotics.

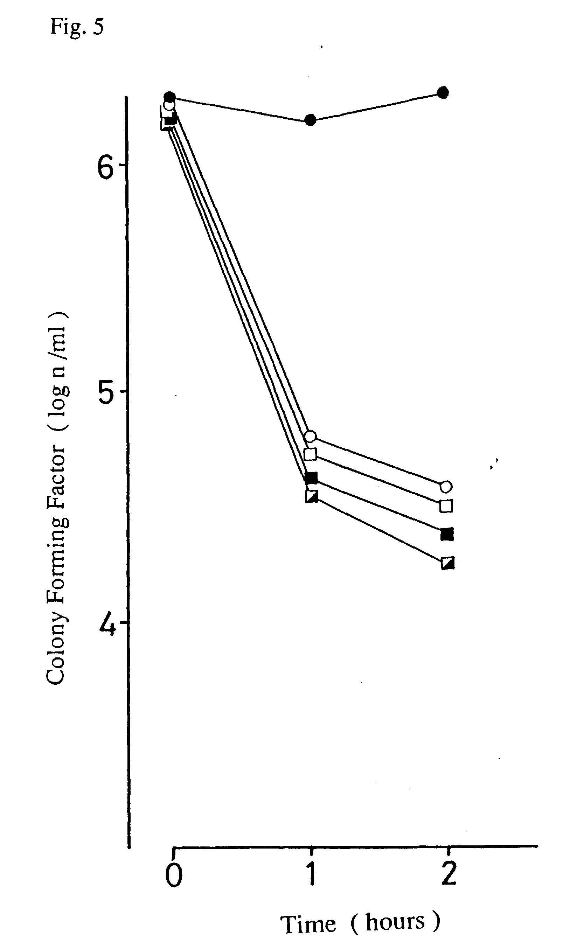

Experimental Example 3-Peritoneal cells were collected from exudate inducted by intraperitoneal injection of starch. Then the macrophages were removed by adherence to the glass surface, and remaining neutrophils were washed several times with saline. The washed neutrophils were mixed with opsonized *Escherichia coli c* 11 in the medium RPMI-1640 and incubated for two hours. As shown in Figure 5, the viable cell counts reduced to 1/50 during the incubation period and the addition of bovine lactoferrin had no effect on the phagocytosis at concentrations of 10, 100 and 1,000 mg/ml.

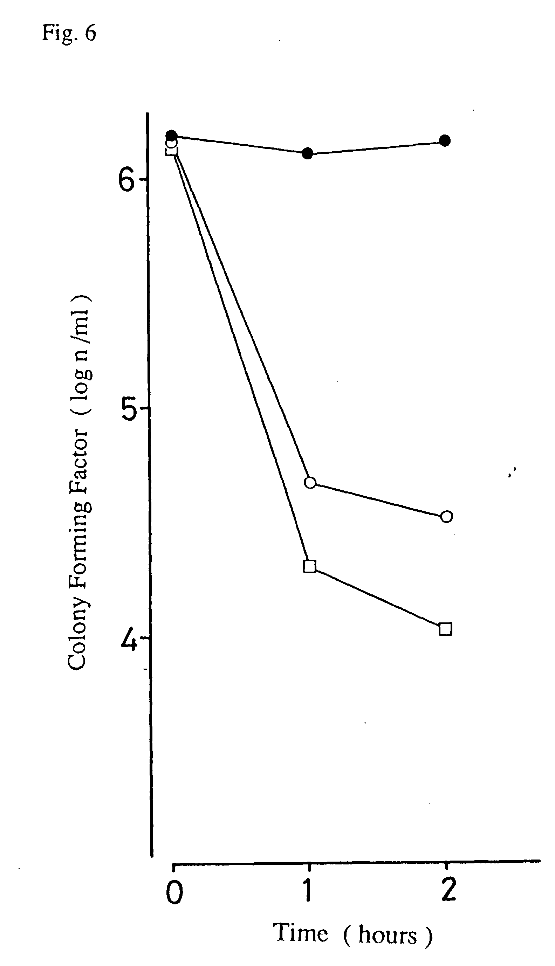

Example 8-Bovine lactoferrin was orally given to male ddY mice, five weeks old, once daily for five consecutive days at a dose of 0.5 mg per mouse. Then they were sacrificed and the blood was withdrawn from the heart. The whole blood was added to the incubation mixture containing opsonized *Escherichia coli c* 11. The viable cells were counted at 0, 1 and 2 hours later. As shown in Figure 6, the clearance of viable cells from the incubation mixture was accelerated two times faster than the controls in the whole blood from lactoferrin-dosed mice.

Example 9-Male ICR mice, five weeks old, were dehydrated overnight, and *Proteus mirabilis* strain 1287 was inoculated into the bladder at a dose of 2 x 10⁵ CFU per mouse. In order for the pathogen to colonize the kidneys, the urethra was closed by a clip for four hours. Ampicillin was orally given to the mice twice daily (10:00 am and 7:00 pm) for two consecutive days with or without oral lactoferrin therapy. On day 3 all mice were sacrificed and the kidneys were dissected ascetically. The kidneys were homogenized in sterile saline and the homogenate was spread over nutrient agar plates to determine the numbers of colonizing bacteria. As shown in Table 6, simultaneous administration of lactoferrin (1 mg per mouse) with ampicillin significantly reduced colonized renal pathogen.

**Table 6.**

| Effect of lactoferrin against upper urinary tract infection | | | |
|---|---|---|---|
| ampicillin (mg/mouse) | colony quantity (CFU/kidney) | | |
| | Lactoferrin (-) | Lactoferrin (+) | % reduction |
| 1.0 | 88 | 0 | <1/88 |
| 0.1 | 63,216 | 36 | 1/1756 |
| 0.01 | 100,704 | 58 | 1/1736 |
| 0.000 | 138,508 | 854 | 1/162 |

Example 10-The effect of oral treatment of lactoferrin on phagocytic activity of neutrophils from peripheral blood was examined using healthy volunteers in a single blind fashion. Eight healthy volunteers, averaged ages ± standard deviation = 47.5 ± 5.3, were randomly allocated into two groups, one group was orally administered granules of bovine native lactoferrin (500 mg as pure lactoferrin, daily) for three consecutive days, and the other group was given placebo (colored skim milk powder). On day 4, blood (15 ml) was withdrawn and the neutrophils were separated using the Ficol density-gradient method. The washed neutrophils were added to the incubation mixture containing opsonized *E. coli* c-11 and disappearance of viable cells from the incubation mixture was determined. As shown in Table 9, lactoferrin treatment significantly accelerated phagocytosis of neutrophils derived from the volunteers, as compared with placebo treated controls.

**Table 7.**

| Effect to increase phagocytic activity of human neutrophils by bovine lactoferrin | | | | |
|---|---|---|---|---|
| trials | volunteer group | phagocytic activity (mean±SD) | | |
| | | Lactoferrin | 1 hour | 2 hours |
| 1st | A | + | 77.8 ± 8.2 | 95.2 ± 10.5 |
| | B | - | 43.3 ± 3.9 | 73.7 ± 6.0 |
| 2nd | A | + | 48.4 ± 4.2 | 65.5 ± 7.8 |
| | B | - | 80.6 ± 6.7 | 88.3 ± 11.0 |
| (n=4) | | | | |

The above experiments show that oral administration of the class lactoferrin proteins to animals results in (1) enhancing the *in vivo* efficacy of bacteriostatic antimicrobial chemotherapies (including antibiotics) by 2-5 fold and (2) allows treatment and/or prevention of opportunistic-infectious diseases in compromised hosts. The mechanism of action of oral lactoferrin therapy is host-mediated activation of neutrophils potentiating host-defense system and efficiently protecting the host from pathogenic microbial attack.

Further variations and modifications of the foregoing will be apparent to those skilled in the art and such variations and modifications are attended to be encompassed by the claims that are appended hereto.

Japanese Priority Application 152620/92 filed on April 2, 1992 is relied on and incorporated by reference.

U.S. Patent Application Serial No. 07/769,766, filed on October 4, 1991, now U.S. Patent No. , and U.S. Patent Application Serial No. 07/624,229, filed on December 7, 1990, are incorporated by reference. The U.S. Patent Application filed on March 1, 1993 entitled "Formulated Medicine For Treatment And Prevention Of Opportunistic Infectious Diseased Complicated By Infection With Lentivirus" (inventors Kunio Ando and Junichi Kishimoto) is also incorporated by reference.

## Claims

1. A pharmaceutical composition for potentiating the immune system of an animal subject comprising (a) proteins belonging to the class of the mammalian transferrin/lactoferrin family and (b) a bacteriostatic agent selected from macrolide, tetracycline antibiotics and sulphonamides, optionally together with a pharmaceutically acceptable carrier.

2. A composition containing (a) proteins belonging to the class of the mammalian transferrin/lactoferrin family and (b) a bacteriostatic agent selected from macrolide, tetracycline antibiotics and sulphonamides antibiotic, as a combined preparation for simultaneous, separate or sequential use in potentiating the immune system of a mammalian subject.

3. A composition as claimed in claim 1 or claim 2, wherein said component (a) is present at a dosage of 0.1 to 250 mg/kg body weight.

4. A composition as claimed in any one of claims 1 to 3, wherein said protein is present at a dosage of 1 to 50 mg/kg body weight.

5. A composition as claimed in any one of claims 1 to 4, in the form of a tablet or a hard gelatin capsule.

6. A composition s claimed in any one of claims 1 to 5 wherein said component (a) is lactoferrin obtainable from mammalian milk, transferrin obtainable from mammalian or avian blood, or ovo-transferrin obtainable from avian albumen.

7. A composition as claimed in any one of claims 1 to 6 wherein said protein belongs to mammalian transferrin/ lactoferrin family independent of its iron saturation including either holotype, or Fe³⁺ iron-saturated, or iron-free apo-type.

8. A composition as claimed in any one of claims 1 to 7 wherein said bacteriostatic agent comprises at least one member selected from erythromycin, chloramphenicol and tetracycline.

9. A food product comprising a food ingredient together with (a) a protein of the class of the mammalian transferrin/lactoferrin family and (b) a bacteriostatic agent, as defined in any one of claims 1 to 8.

10. A food product as claimed in claim 9, wherein said lactoferrin is present in an amount over 150 mg/kg body weight and said food product is solid.

11. The food product according to claim 9, wherein said lactoferrin is present in an amount over 30 mg/ml and said food product is liquid.

12. A pharmaceutical composition for potentiating the immune system of a mammalian subject comprising (a) proteins belonging to the class of the mammalian transferrin/lactoferrin family and (b) a bacteriostatic agent, optionally together with a pharmaceutically acceptable carrier.

13. A composition containing (a) proteins belonging to the class of the mammalian transferrin/lactoferrin family and (b) a bacteriostatic agent, as a combined preparation for simultaneous, separate or sequential use in potentiating the immune system of a mammalian subject.

14. A composition as claimed in claim 12 or claim 13 for potentiating the immune system against opportunistic infectious diseases in mammals.

15. The composition as claimed in any one of claims 12 to 14, wherein said protein is lactoferrin obtainable from mammalian milk, transferrin obtainable from mammalian or avian blood, or ovo-transferrin obtainable from avian albumen.

16. A composition as claimed in any one of claims 12 to 15 wherein said protein belongs to mammalian transferrin/lactoferrin family independent of its iron saturation including either holotype, or Fe³⁺ iron-saturated, or iron-free apo-type.

17. A composition as claimed in any one of claims 12 to 16, wherein said protein is present at a dosage of 0.1 to 250 mg/kg body weight.

18. A composition as claimed in any one of claims 12 to 17, wherein said protein is present at a dosage of 1 to 50 mg/kg body weight.

19. A composition as claimed in any one of claims 12 to 18, wherein said bacteriostatic agent comprises at least one member selected from β-lactam, macrolide, and tetracycline antibiotics and sulphonamides.

20. A composition as claimed in any one of claims 12 to 19, wherein said bacteriostatic agent comprises at least one member selected from cephalexin, cefaclor, cephroxazine, cefixime, ampicillin, erythromycin, chloramphenicol, and tetracycline.

21. A composition as claimed in any one of claims 12 to 20, in the form of a tablet or a hard gelatin capsule.

22. A food product comprising a food ingredient together with (a) a protein of the class of the mammalian transferrin/lactoferrin family and (b) a bacteriostatic agent, as defined in any one of claims 12 to 20.

23. A food product as claimed in claim 22, wherein said protein is present in an amount over 150 mg/kg body weight and said food product is solid.

24. The food product according to claim 22, wherein said lactoferrin is present in an amount over 30 mg/ml and said food product is liquid.

25. Use of a protein belonging to the class of the mammalian transferrin/lactoferrin family as defined in any one of claims 12 to 18 for the preparation of a medicament or medicated food product for use together with a bacteriostatic agent as defined in any one of claims 12, 19 and 20 in potentiating the immune system of a mammalian subject.

26. Use of a bacteriostatic agent as defined in any one of claims 12, 19 and 20 for the preparation of a medicament or medicated food product for use together with a protein belonging to the class of the mammalian transferrin/lactoferrin family in potentiating the immune system of a mammalian subject.
